# EUROPÄISCHE PATENTSCHRIFT

(11) **EP 1 382 291 B1**
(45) Veröffentlichungstag und Bekanntmachung des Hinweises auf die Patenterteilung: **02.12.2009**
(21) Anmeldenummer: 03090226.6
(22) Anmeldetag: 18.07.2003
(51) Int. Cl.: A61B 1/12, A61B 17/32, A61M 1/00

(54) **Vorrichtung zum Durchspülen einer Körperhöhle**
Device for irrigation of a body cavity
Dispositif d'irrigation d'une cavité corporelle

(30) Priorität: 19.07.2002 DE 10233053
(43) Veröffentlichungstag der Anmeldung: 21.01.2004
(73) Patentinhaber: W.O.M. World of Medicine AG, 96337 Ludwigsstadt (DE)
(72) Erfinder: Haischmann, Fabian, 10553 Berlin (DE); Merzhaeuser, Thomas, 10553 Berlin (DE); Stiller, Matthias, 10553 Berlin (DE); Reuther, Martin, 10553 Berlin (DE)
(74) Vertreter: Jungblut, Bernhard Jakob

(56) Entgegenhaltungen:
- US-A- 5 630 799
- US-A- 5 931 808
- US-A- 6 024 720

## Beschreibung

### Gebiet der Erfindung.

Die Erfindung betrifft eine Vorrichtung zum Durchspülen einer Körperhöhle mit einem Fluid, mit einem Vorratsbehälter für das Fluid, mit einer an den Vorratsbehälter angeschlossenen, ansteuerbaren Spülpumpe, mit einer an eine Druckseite der Spülpumpe angeschlossenen Zuführleitung sowie mit einem an die Zuführleitung angeschlossenen ersten ärztlichen Instrument mit einem Spülkanal, welches in die Körperhöhle einführbar ist, wobei druckseitig der Spülpumpe ein Drucksensor zur Bestimmung eines Ist-Druckes eingerichtet ist, mit einer in die Körperhöhle einführbaren Drainagekanüle mit Drainageleitung, mit einem in die Körperhöhle einführbaren zweiten ärztlichen Instrument mit Absaugleitung, wobei ein durch die Körperhöhle einstellbarer Volumenstrom nach Maßgabe einer Betriebsstellung "an" oder "aus" des zweiten ärztlichen Instrumentes "hoch" oder "niedrig" ist, wobei die Drainagekanüle sowie die Absaugleitung an eine Saugpumpe angeschlossen sind, ein Verfahren zum Betrieb einer solchen Vorrichtung sowie eine Verwendung einer solchen Vorrichtung.

Solche Vorrichtungen werden beispielsweise für endoskopische Untersuchungen, Distension oder Resektion von Geweben, insbesondere unter endoskopischer Kontrolle, eingesetzt. Eine Körperhöhle kann ein Gelenkhohlraum, z.B in einem Kniegelenk oder Schultergelenk, ein Hohlraum zwischen Muskeln oder Organen, oder auch ein einen Holhraum bildendes oder aufweisendes Organ selbst sein. Ein Fluid ist insbesondere ein Flüssigkeit. Dabei kann es sich um eine homogene flüssige Phase oder auch um eine Dispersion oder Emulsion handeln. Eine Drainagekanüle im Sinne der Erfindung kann neben der Drainagefunktion an sich auch weitere Funktionen erfüllen, wie beispielsweise ein Ausleuchten der Körperhöhle über eine in die Drainagekanüle integrierte Lichtquelle. Unterschiedliche Volumenströme bei unterschiedlichen Betriebsstellungen sind im Rahmen der Erfindung durch die Begriffe "hoch" und "niedrig" unterschieden, wobei die absoluten Werte irrelevant sind. Vielmehr wird mit diesen Begriffen lediglich zum Ausdruck gebracht, wie die Volumenströme relativ zueinander bei den jeweiligen Betriebstellungen des zweiten ärztliche Instruments sind.

Stand der Technik.

Vorrichtungen des eingangs genannten Aufbaus sind beispielsweise aus den Literaturstellen US-4,902,277, US-5,000,733 und EP-0 306 445 bekannt. Die Umschaltung der Absaugleistung auf fest voreingestellte Werte erfolgt durch Signalkontakte einer Bedienungseinheit für einen Shaver und Verwendung dieses Signals zur Steuerung einer Saug-Spülvorrichtung. Es erfolgt eine gleichzeitige Umschaltung der Absaugleistung zwischen Shaver und Drainagekanüle. Nachteilig bei der insofern bekannten Vorrichtung ist, daß bei Verwendung eines Shavers dem System von den Signalkontakten, typischerweise ein Fußschalter, die Betriebseinstellungen "an" und "aus" übermittelt werden müssen. Dies kann im einfachsten Fall durch Signalleitungen mit Steckverbindungen erfolgen. Dies ist aufwendig insbesondere im Aufbau. Des weiteren bestehen funktionelle Fehlermöglichkeiten, beispielsweise durch schlechte Kontakte aufgrund von Kontaktkorrosion und dergleichen. Schließlich unterliegen die Steckverbindungen durch vielfache Betätigung einem störenden Verschleiß. Ein besonders bedeutender Nachteil ist, dass der Shaver kompatibel zur Vorrichtung des eingangs genannten Aufbaus sein muß, da eine Signalisierung ausschließlich die digitale Information "an" und "aus" unfaßt und folglich die Steuerung in einer Steuereinheit jeweils auf das verwendete zweite ärztliche Instrument, beispielsweise der Shaver, eingestellt werden muß. Mangelt es einer solchen Einstellbarkeit, kann nur ein bestimmtes ärztliches Instrument an der Steuereinheit betrieben werden. Es mangelt im Ergebnis einer Kompatibilität zwischen einer einzigen Steuereinheit und mehreren verschiedenen ärztlichen Instrumenten und/oder gleichen ärztlichen Instrumenten verschiedener Hersteller und verschiedener Spezifikationen hinsichtlich des Strömungswiderstandes. Schließlich ist beim Stand der Technik nachteilig, daß mit der Signalisierung von Betriebsstellung "aus" zu "an" des zweiten ärztlichen Instrumentes körperhöhlenzuführseitig eine sofortige Hochregelung des Flusses erfolgt, während die erhöhte Abfuhr des Fluides durch das zweite ärztliche Instrument vergleichsweise langsam "hochfährt". Dadurch entsteht das Risiko eines medizinisch störenden Druckpeaks in der Körperhöhle.

Eine Vorrichtung gemäß dem Oberbegriff des Anspruchs 1 ist aus der US-6,024,277 bekannt.

### Technisches Problem der Erfindung.

Der Erfindung liegt das technische Problem zu Grunde, eine Vorrichtung zum Durchspülen einer Körperhöhle mit einem Fluid anzugeben, mittels welcher eine allen Anforderungen genügende Steuerung von Fluss und Druck erzielt wird, und zwar ohne das Erfordernis von Signalleitungen und unabhängig von dem eingesetzten zweiten ärztlichen Instrument.

### Grundzüge der Erfindung und bevorzugte Ausführungsformen

Zur Lösung dieses technischen Problems lehrt die Erfindung im Kennzeichen des Anspruchs 1, daß allein die Drainageleitung mit ansteuerbaren Mitteln zur Variation des Durchflußwiderstandes ausgestattet ist, daß Spülpumpe, Drucksensor und Mittel zur Variation des Durchflußwiderstandes an Steuerein- oder -ausgänge einer Steuereinheit angeschlossen sind, wobei mittels der Steuereinheit ein Abfall des mittels des Drucksensors gemessenen Ist-Druckes von einem vorgegebenen Soll-Druck bei Umschaltung des zweiten ärztlichen Instrumentes aus der Betriebsstellung "aus" zu "an" detektierbar und in ein Steuersignal für die Mittel zur Variation des Durchflußwiderstandes, die Spülpumpe und/oder die Saugpumpe umwandelbar ist, und zwar nach Maßgabe einer voreingestellten Soll-Förderleistung der Spülpumpe und/oder der Saugpumpe für die Betriebsstellung "an" sowie des Soll-Druckes, und umgekehrt.

Mit anderen Worten ausgedrückt, in beispielsweise der Betriebsstellung "aus" eines Shavers erfolgt die Absaugung des Fluids aus der Körperhöhle im wesentlichen über die Drainageleitung. Mit der Umschaltung des Shavers in die Betriebstellung "ein" wird gleichsam ein Bypass, nämlich die Absaugleitung zur Drainageleitung aktiviert mit der Folge eines (kurzzeitigen) Druckabfalls in der Körperhöhle. Dieser Druckabfall wird detektiert mit der Folge, daß der Fluss durch Ansteuerung der Spülpumpe auf einen vorgewählten (höheren als bei Shaver "aus") Wert heraufgefahren wird. Die Druckregelung kann dann bei konstantem Fluss mittels Ansteuerung der Saugpumpe erfolgen.

Mit der Erfindung wird erreicht, daß der Fluss und Druck in der Körperhöhle unabhängig voneinander frei regelbar und konstant haltbar sind, und zwar unabhängig von der Betriebsstellung des zweiten ärztlichen Instruments. Die Regelung des Flusses erfolgt mit den erfindungsgemäßen Mitteln zur Variation des Durchflußwiderstandes schneller als mit einer drehzahlgeregelten Rollenpumpe aufgrund der geringeren Massen und Trägheitsmomente der Mittel zur Variation des Durchflusswiderstandes. Die Anpassung des Flusses und ggf. Einstellung oder Nachregelung des Druckes erfolgt nach der Maßgabe der Betriebseinstellung "an" oder "aus" des zweiten ärztlichen Gerätes. Vorteilhaft ist auch, dass in der Körperhöhle auftretende Druckschwankungen vom System innerhalb sehr kurzer Zeit erkannt und auf den Soll-Wert des Druckes korrigiert werden, so dass der Druck in der Körperhöhle auch bei Umstellungen zwischen den Betriebstellungen des zweiten ärztlichen Instrumentes praktisch konstant gehalten wird. Ein weiterer Vorteil ist, dass jedes ärztliche Instrument ohne aufwendige Adaptierung von Signalleitungen und Steckverbindungen verwendet werden kann, da Steuerungs- und Regelungsmechanismen in jedem Fall für eine Einstellung und Konstanthaltung der voreingestellten Werte für Druck und Fluß sorgen. Schließlich ist die Anordnung der Mittel zur Variation des Durchflußwiderstandes im Rahmen der Drainageleitung sicherheitstechnisch vorteilhaft, weil die Druck- und Flussregelung bei Einschaltung des ärztlichen Instrumentes gleichsam nachläuft, wodurch gesundheitlich bedenkliche Druckspitzen vermieden werden. Gleichzeitig werden eventuelle Leckagen durch das Nachlaufen der Regelung automatisch und ohne zusätzlich Maßnahmen berücksichtigt und ausgeglichen. Letztendlich wird eine praktisch konstante Körperhöhlenausdehnung und entsprechend gute Sicht durch eine endoskopische Optik erreicht.

Das erste ärztliche Instrument kann ausgewählt sein aus der Gruppe bestehend aus "Spülsonde, Spülkanüle, Trokar mit Optik und Optik mit Spülkanal"

Die Spülpumpe kann eine Antriebseinheit mit einem Motor mit rotierender Abtriebswelle sowie eine mit der Abtriebswelle (mechanisch, i.e. stoffschlüssig, formschlüssig oder kraftschlüssig, oder magnetisch, i.e. kraftschlüssig) verbundene und rotierend angetriebene Pumpeneinheit aufweisen, wobei die Förderleistung durch Ansteuerung der Drehzahl des Motors ansteuerbar ist. Insbesondere kommt insofern eine peristaltische Rollenpumpe in Frage. Alternativ kann die Spülpumpe einen elastischen Vorratsbehälter mit einer den Vorratsbehälter teilweise odder ganz ummantelnden ansteuerbaren Druckmaschette aufweisen, wobei die Förderleistung durch Ansteuerung der Druckmanschette ansteuerbar ist. Schließlich kann weiterhin alternativ die Spülpumpe einen höhenansteuerbaren Vorratsbehälter aufweisen, wobei die Förderleistung durch Ansteuerung der Höhe des Vorratsbehälters ansteuerbar ist.

Das zweite ärztliche Instrument kann ausgewählt sein aus der Gruppe bestehend aus "Shaver, Probenentnahmegerät und Saugsonde". Insbesondere im Falle eines Shavers empfiehlt es sich, daß der Volumenstrom durch das zweite ärztliche Instrument in der Betriebsstellung "an" "hoch" ist und umgekehrt. Es ist aber ggf. auch der umgekehrte Zusammenhang möglich.

Grundsätzlich können die Steuereinheit sowie die hiermit verbundenden Komponenten hydraulisch, pneumatisch oder elektrisch betrieben bzw. angesteuert werden. Bevorzugt ist der elektrische bzw. elektromechanische Betrieb.

Die Mittel zur Variation des Durchflußwiderstandes können proportional, kontinuierlich oder in einer Mehrzahl von diskreten Schritten, ansteuerbar sein. Bevorzugt ist es, dass die Mittel zur Variation des Durchflußwiderstandes eine Schlauchabklemmvorrichtung mit einem Schlauch sind. Die Schlauchwandung des Schlauches besteht dann aus einem elastischen Werkstoff. Im Einzelnen können die Mittel zur Variation des Durchflußwiderstandes mit einer Stützfläche, auf welcher die Schlauchwandung des Schlauches aufliegt, aufweisen und mit einem Druckstück ausgestattet sein, mittels welchem auf die Schlauchwandung Druck in Richtung der Stützfläche ausgeübt werden kann. Bevorzugt ist, daß das Druckstück linear antreibbar und über ein Spindelgetriebe mit einem elektromotorischen Antrieb, vorzugsweise einem Schrittschaltmotor, verbunden ist. Je nach gewünschter Steuer- oder Regelcharakteristik können aber auch nichtlineare Funktionen zwischen einem Steuersignal und der Bewegung des Druckstückes eingerichtet sein. Beispiel hierfür wäre eine rotierend linear angetriebene runde Exzenterscheibe, deren Mantelfläche gegen die Schlauchwandung oder ein Druckstück läuft. Mittels einer Nockenscheibe lassen sich beliebige Kinematiken einrichten.

Es wird sich empfehlen, daß die Drainageleitung und die Absaugleitung bzw. die Saugpumpe letztendlich in ein Auffanggefäß münden.

Die Erfindung lehrt desweiteren ein Verfahren zum Betrieb einer erfindungsgemäßen Vorrichtung, wobei bei Umschaltung eines zweiten ärztlichen Instrumentes zwischen zwei Betriebsstellungen, welche mit unterschiedlichem Fluß durch die Absaugleistung korreliert sind, mittels des Drucksensors und der Steuereinheit eine Druckveränderung detektiert wird, wobei mittels der Steuereinheit auf Detektion einer Druckveränderung die Mittel zur Variation des Durchflußwiderstandes angesteuert werden nach Maßgabe der Einregelung auf einen vorgegebenen und der aktiven Betriebstellung des zweiten ärztlichen Instrumentes zugeordneten Soll-Fluss und/oder Soll-Druck. Regelungstechnisch wird es sich empfehlen, daß auf Detektion einer Druckveränderung zusätzlich die Spülpumpe und/oder die Saugpumpe von der Steuereinheit angesteuert werden nach Maßgabe der Einregelung auf einen vorgegebenen und der aktiven Betriebstellung des zweiten ärztlichen Instrumentes zugeordneten Soll-Fluss und/oder Soll-Druck.

Im Folgenden wird die Erfindung anhand von lediglich ein Ausführungsbeispiel darstellenden Figuren näher erläutert.

Es zeigen:
- Fig. 1:: eine schematische Gesamtdarstellung einer erfindungsgemäßen Vorrichtung
- Fig. 2:: eine Detaildarstellung zu einem baulich besonders einfachen Mittel zur Variation des Durchflußwiderstandes und
- Fig. 3:: eine schematische Darstellung eines erfindungsgemäß eingesetzten Schlauchsets sowie dessen Anordnung.

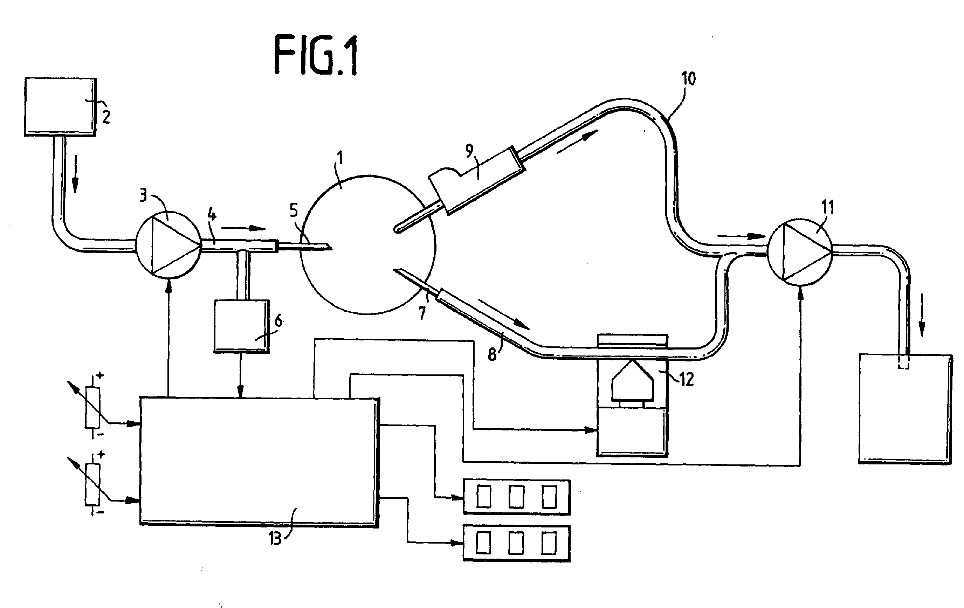

In der Figur 1 erkennt man eine Vorrichtung zum Durchspülen einer Körperhöhle 1 mit einem Fluid. Diese Vorrichtung ist mit einem Vorratsbehälter 2 für das Fluid und mit einer an den Vorratsbehälter 2 angeschlossenen, ansteuerbaren Spülpumpe 3 ausgestattet. An einer Druckseite der Spülpumpe 3 ist eine Zuführleitung 4 angeschlossenen. Die Zuführleitung 4 ist mit einem ersten ärztlichen Instrument mit einem Spülkanal 5, welches in die Körperhöhle 1 einführbar ist, versehen. Druckseitig der Spülpumpe 3 ist ein Drucksensor 6 zur Bestimmung eines Ist-Druckes eingerichtet.

Die dargestellte Ausführungsform ist mit einer in die Körperhöhle 1 einführbaren einfachen Drainagekanüle 7 mit Drainageleitung 8 und mit einem in die Körperhöhle 1 einführbaren zweiten ärztlichen Instrument 9 mit Absaugleitung 10 ausgestattet, wobei ein durch die Körperhöhle 1 einstellbarer Volumenstrom nach Maßgabe einer Betriebsstellung "an" oder "aus" des zweiten ärztlichen Instrumentes 9 "hoch" oder "niedrig" ist. Die Drainagekanüle 7 sowie die Absaugleitung 10 sind mit einer Saugpumpe 11 verbunden. Der Drainageleitung 8 enthält ansteuerbare Mittel zur Variation des Durchflußwiderstandes 12. Sowohl Spülpumpe 3, Drucksensor 6 als auch Mittel zur Variation des Durchflußwiderstandes 12 sind an Steuerein- oder -ausgänge einer Steuereinheit 13 angeschlossen.

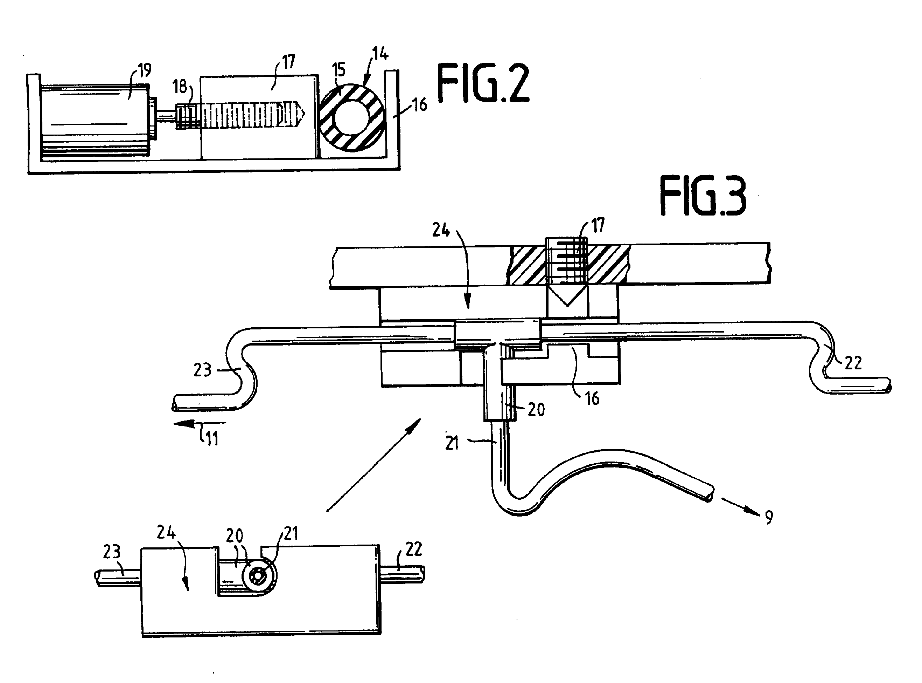

In der Figur 2 erkennt man ein baulich besonders geeignetes Mittel zur Variation des Durchflußwiderstandes 12. Es handelt sich um eine Schlauchabklemmvorrichtung mit einem Schlauch 14, dessen Schlauchwandung 15 aus einem elastischen Werkstoff besteht. Die Schlauchabklemmvorrichtung weist eine Stützfläche 16 auf, auf welcher der Schlauch aufliegt 14, und ein Druckstück 17, mittels welchem auf die Schlauchwandung 15 Druck in Richtung der Stützfläche ausübbar ist. Das Druckstück 17 ist über ein Spindelgetriebe 18 mit einem elektromotorischen Antrieb 19 verbunden.

Folgend wird die Betriebsweise dieser Ausführungsform der Erfindung näher erläutert. Die Saugpumpe 11 läuft mit einer Saugleistung, die bei Verwendung eines Shavers 9 dem in dessen Betriebstellung "an" entsprechenden Fluss entspricht, und wird in der Betriebsstellung "aus" von der Regelung 13 durch proportional und kontinuierlich regelbare Schlauchabklemmung 12 auf die gewünschte Drainageleistung reduziert. In Betriebsstellung "an" entsteht durch die Absaugleitung 10 über den Shaver ein Bypass, welcher an der Drainageleitung 8 vorbei führt. Der Körperhöhleninnendruck nimmt aufgrund der Addition der beiden Volumenströme durch Drainageleitung 8 und Absaugleitung 10 ab. Dieser Druckabfall wird vom Drucksensor 6 sehr schnell registriert. Die an den Drucksensor 6 angeschlossene Steuereinheit 11 detektiert den Abfall und erkennt hierdurch die Betriebsstellung "an" des Shavers und steuert über ein Steuersignal die Schlauchabklemmeinrichtung 12 an, welche die Drainageleitung 8 abklemmt. Gleichzeitig wird die Spülpumpe 3 auf eine Erhöhung der Pumpleistung auf einen vorgewählten Wert angesteuert. Durch Ansteuerung der Saugpumpe 11 erfolgt die Druckregelung durch Änderung der Pumpleistung nach Maßgabe des vorgewählten Druckes, welcher grundsätzlich und unabhängig von dieser konkreten Ausführungsform auch unterschiedliche vorgewählte Werte für die Betriebsstellungen des zweiten ärztlichen Instrumentes aufweisen kann. Wird der Shaver ausgeschaltet, steigt aufgrund des fehlenden Abflußstroms durch die Absaugleitung 10 der Körperhöhleninnendruck kurzzeitig an. Der Anstieg des Druckes über den vorgewählten Innendruck wird ebenfalls vom Drucksensor 6 registriert; es erfolgen die umgekehrten Prozesse, wie vorstehend beschrieben. Im Betriebszustand "aus" des Shavers kann der Druck über die Pumpleistung der Spülpumpe 3, über die Pumpleistung der Saugpumpe 11 und/oder über die Schlauchabklemmvorrichtung 12 geregelt werden.

Unabhängig von der vorstehenden Ausführungsform kann eine Bestimmung des wahren Innendruckes in der Körperhöhle 1 dadurch erfolgen, daß die Spülpumpe 3 und die Saugpumpe 11 angehalten werden, wobei der vom Drucksensor 6 erhaltene Messwert aufgrund der statischen Verhältnisse (Fluss ist praktisch null) dem wahren Innendruck entspricht. Dies kann alternierend mit der vorstehend beschriebenen erfindungsgemäßen Betriebsweise erfolgen. Des Weiteren ist es möglich, dass Meßwertunterschiede des Drucksensors in den insofern durchgeführten Intervallen (Fluss = voreingestellt / Fluss = 0) für eine interne Kalibrierung und Abstimmung auf das erste ärztliche Instrument 5 bzw. des Druckabfalls hierüber genutzt werden. Dann wird die Steuereinheit 13 diese Unterschiede berücksichtigen bei der vorstehend beschriebenen Regelung auf einen voreingestellten Soll-Druckwert, i.e. auf einen dengegenüber entsprechend höheren Messwert des Drucksensors 6 einregeln. Dies kann ggf. auch als im Wege einer nach Anschluss eines spezifischen ersten ärztlichen Instrumentes 5 bestimmten, gleichsam "erlernten" und in der Steuereinheit 13 abgespeicherten Kalibrierungskurve erfolgen. Es wird sich empfehlen, das "Erlernen" nach Anschluss eines ersten ärztliche Instrumentes und vor oder zu Beginn einer Behandlung durchzuführen.

Figur 3 zeigt ein Schlauchset für eine erfindungsgemäße Vorrichtung mit einem T-Stück 20 und an das T-Stück 20 angeschlossenen Schläuchen 21,22,23, wobei ein erster Schlauch 21 zum Anschluß an ein zweites ärztliches Instrument 9 bestimmt ist, wobei ein zweiter Schlauch 22 zumindest einen Teil der Drainageleitung 8 bildet, wobei ein dritter Schlauch 23 zum Anschluß an die Saugpumpe 11 bestimmt ist, wobei optional der erste Schlauch 21 und der dritte Schlauch 23 aus einem Polymer einer Härte von zumindest Shore A 70, vorzugsweise 75, und der zweite Schlauch 22 aus einem Polymer einer Härte von weniger als Shore A 70, vorzugsweise 65, gebildet ist. Das T-Stück ist 20 aus einem formstabilen Kunststoff gebildet und formschlüssig komplementär zu einer in baulicher Einheit mit einem Mittel zur Variation des Durchflusswiderstandes 12 eingerichteten T-Stück-Formschlussausnehmung 24 ausgebildet. Die bauliche Einheit aus Mittel zur Variation des Durchflußwiderstandes 12 und T-Stück-Formschlussausnehmung 24 ist ggf. lösbar an einer Gerätewandung befestigt, welche wiederum die Antriebsmittel für das Druckstück 17 trägt womit das Druckstück 17 gegen die Stützfläche 16 antreibbar und so den zweiten Schlauch 22 klemmend ist. Es versteht sich, daß der zweite Schlauch 22 zumindest im Bereich zwischen dem Druckstück 17 und der Stützfläche die vorstehend beschriebene niedrigere Shore Härte aufweist. Das Einlegen des T-Stückes 20 in die T-Stückformschlussausnehmung 24 und die Fixierung hierin ergeben sich unmittelbar aus einer vergleichenden Betrachtung der Teilfiguren der Figur 3.

Im Folgenden wird ein alternatives Verfahren zum Betrieb einer erfindungsgemäßen Vorrichtung beschrieben. Die Saugpumpe 11wird mit einer Saugkapazität (Fluss, Geschwindigkeit, Drehzahl) betrieben, welche einstellbar und folglich vorbestimmt ist auf einen Wert, welcher zum Betrieb des zweiten ärztlichen Instrumentes 9, beispielsweise eines Shavers 9, angemessen ist, jedoch nicht in Abhängigkeit von dem Druck in der Körperhöhle 1 geregelt und/oder gesteuert wird. Die Saugpumpe 11 wird vielmehr durch die Steuereinheit 13 kontinuierlich mit einer Geschwindigkeit angetrieben, die dem gewünschten Fluss durch das zweite ärztliche Instrument 9 in dessen aktiviertem Betriebszustand entspricht. Dadurch wird in den Schläuchen zwischen der Saugpumpe 11, dem zweiten ärztlichen Instrument 9 und den Mitteln zur Variation des Durchflusswiderstandes 12 ein Unterdruck erzeugt. Wenn nunmehr das zweite ärztliche Instrument 9 aktiviert wird, steigt der Fluss durch das zweite ärztliche Instrument 9 von null oder einem Minimalwert auf den Betriebswert an, wobei der Betriebswert des Flusses von der Einstellung der Geschwindigkeit der Saugpumpe 11, wie von einer Bedienperson eingestellt oder durch Programmierung der Steuereinheit 13 bestimmt, abhängt. Der Anstieg des Flusses durch das zweite ärztliche Instrument 9 (wobei die Position des Mittels zur Variation des Durchflusswiderstandes 12 zumindest anfänglich unverändert ist) führt zu einem Druckabfall in der Körperhöhle 1. Sobald der Druckabfall mittels des Drucksensors 6 detektiert wird, erhöht die Steuereinheit 13 den Fluss durch die Spülpumpe, beispielsweise durch Erhöhung der Geschwindigkeit/Drehzahl, um den Druckabfall zu kompensieren. Wenn durch Erhöhung des Flusses durch die Spülpumpe eine Kompensation (i.e. Einstellung auf den voreingestellten und zuvor eingeregelten Sollwert) nicht erreichbar ist, steuert die Steuereinheit 13 zusätzlich die Mittel zur Variation des Durchflusswiderstandes 12 zur Erhöhung des Durchflusswiderstandes an, wodurch der gesamte Fluss aus der Körperhöhle 1 zur Saugpumpe 11 reduziert wird. Letzteres setzt selbstverständlich voraus, dass die Mittel zu Variantion des Durchflusswiderstandes 12 auf eine Einstellung unterhalb des maximalen Durchflusswiderstandes angesteuert sind, wenn das zweite ärztliche Instrument 9 nicht aktiviert ist.

## Patentansprüche

1. Vorrichtung zum Durchspülen einer Körperhöhle (1) mit einem Fluid,
mit einem Vorratsbehälter (2) für das Fluid, mit einer an den Vorratsbehälter (2) angeschlossenen, ansteuerbaren Spülpumpe (3), mit einer an eine Druckseite der Spülpumpe (3) angeschlossenen Zuführleitung (4) sowie mit einem an die Zuführleitung (4) angeschlossenen ersten ärztlichen Instrument (5) mit einem Spülkanal, welches in die Körperhöhle (1) einführbar ist, wobei druckseitig der Spülpumpe (3) ein Drucksensor (6) zur Bestimmung eines Ist-Druckes eingerichtet ist,
mit einer in die Körperhöhle (1) einführbaren Drainagekanüle (7) mit Drainageleitung (8),
mit einem in die Körperhöhle (1) einführbaren zweiten ärztlichen Instrument (9) mit Absaugleitung (10), wobei ein durch die Körperhöhle (1) einstellbarer Volumenstrom nach Maßgabe einer Betriebsstellung "an" oder "aus" des zweiten ärztliche Instrumentes (9) "hoch" oder "niedrig" ist,
wobei die Drainageleitung (8) sowie die Absaugleitung (10) an eine Saugpumpe (11) angeschlossen sind,
und der Drucksensor zwischen der Spülpumpe (3) und der Auslassöffnung des ersten ärztlichen Instrumentes(5) außerhalb der Körperhöhle (1) angeschlossen ist,
**dadurch gekennzeichnet,**
**daß** allein die Drainageleitung (8) mit ansteuerbaren Mitteln zur Variation des Durchflußwiderstandes (12) ausgestattet ist, daß Spülpumpe (3), Drucksensor (6), Saugpumpe (11) und Mittel zur Variation des Durchflußwiderstandes (12) an Steuerein- oder -ausgänge einer Steuereinheit (13) angeschlossen sind,
wobei mittels der Steuereinheit (13) ein Abfall des mittels des Drucksensors (6) gemessenen Ist-Druckes von einem vorgegebenen Soll-Druck bei Umschaltung des zweiten ärztlichen Instrumentes (9) aus der Betriebsstellung "aus" zu "an" detektierbar und in ein Steuersignal für die Mittel zur Variation des Durchflußwiderstandes (12), die Spülpumpe (3) und/oder die Saugpumpe (11) umwandelbar ist, und zwar nach Maßgabe einer voreingestellten Soll-Förderleistung der Spülpumpe (3) und/oder der Saugpumpe (11) für die Betriebsstellung "an" sowie des Soll-Druckes, und umgekehrt.

2. Vorrichtung nach Anspruch 1, **dadurch gekennzeichnet, daß** das erste ärztliche Instrument (5) ausgewählt ist aus der Gruppe bestehend aus "Spülsonde, Trokar mit Optik, Optik mit Spülkanal und Spülkanüle"

3. Vorrichtung nach Anspruch 1 oder 2, **dadurch gekennzeichnet, daß** die Spülpumpe (3) eine Antriebseinheit mit einem Motor mit rotierender Abtriebswelle sowie einer Pumpeneinheit aufweist, wobei die Förderleistung durch Ansteuerung der Drehzahl des Motors ansteuerbar ist, oder daß die Spülpumpe (3) einen elastischen Vorratsbehälter mit einer den Vorratsbehälter ummantelnden ansteuerbaren Druckmaschette aufweist, wobei die Förderleistung durch Ansteuerung der Druckmanschette ansteuerbar ist, oder daß die Spülpumpe (3) einen höhenansteuerbaren Vorratsbehälter aufweist, wobei die Förderleistung durch Ansteuerung der Höhe des Vorratsbehälters ansteuerbar ist.

4. Vorrichtung nach einem der Ansprüche 1 bis 3, **dadurch gekennzeichnet, daß** das zweite ärztliche Instrument (9) ausgewählt ist aus der Gruppe bestehend aus "Shaver und Saugsonde".

5. Vorrichtung nach einem der Ansprüche 1 bis 4, **dadurch gekennzeichnet, daß** der Volumenstrom durch das zweite ärztliche Instrument (9) in der Betriebsstellung "an" "hoch" ist und umgekehrt.

6. Vorrichtung nach einem der Ansprüche 1 bis 5, **dadurch gekennzeichnet, daß** die Mittel zur Variation des Durchflußwiderstandes (12) proportional kontinuierlich oder in einer Mehrzahl von diskreten Schritten ansteuerbar sind.

7. Vorrichtung nach einem der Ansprüche 1 bis 6, **dadurch gekennzeichnet, daß** die Mittel zur Variation des Durchflußwiderstandes (12) eine Schlauchabklemmvorrichtung sind mit einem Schlauch (14), dessen Schlauchwandung (15) aus einem elastichen Werkstoff besteht, mit einer Stützfläche (16), auf welcher der Schlauch (14) aufliegt, und mit einem Druckstück (17), mittels welchem auf die Schlauchwandung (15) Druck in Richtung der Stützfläche (16) ausübbar ist.

8. Vorrichtung nach einem der Ansprüche 1 bis 7, **dadurch gekennzeichnet, daß** das Druckstück (17) linear antreibbar ist.

9. Vorrichtung nach Anspruch 8, **dadurch gekennzeichnet, daß** das Druckstück (17) über ein Spindelgetriebe (18) mit einem elektromotorischen Antrieb (19), vorzugsweise einem Schrittschaltmotor, verbunden ist.

## Claims

1. A device for rinsing a body cavity (1) with a fluid,
comprising a storage container (2) for the fluid, comprising a controllable rinsing pump (3) connected to the storage container (2), comprising a feed line (4) connected to a pressure side of the rinsing pump (3) and comprising a first medical instrument (5) connected to the feed line (4) and having a rinsing channel, said first medical instrument (5) being capable of being introduced into the body cavity (1), on the pressure side of the rinsing pump (3) a pressure sensor (6) for determining an actual pressure being provided,
comprising a drainage cannula (7) being capable of being introduced into the body cavity (1) and having a drainage line (8),
comprising a second medical instrument (9) being capable of being introduced into the body cavity (1) and having a suction line (10), an adjustable volume flow through the body cavity (1) being "high" or "low", according to the operating position "on" or "off" of the second medical instrument (9),
the drainage line (8) and the suction line (10} being connected to a suction pump (11),
and the pressure sensor being connected between the rinsing pump (3) and the outlet opening of the first medical instrument (5) outside of the body cavity (1),
**characterized in**
**that** solely the drainage line (8) is provided with controllable means for varying the flow resistance (12),
**that** rinsing pump (3), pressure sensor (6), suction pump (11) and means for varying the flow resistance (12) are connected to control inputs or outputs of a control unit (13),
wherein by means of the control unit (13) a drop of the actual pressure measured by means of the pressure sensor (6) from a given target pressure can be detected when switching the second medical instrument (9) from the operating position "off" to "on" and can be converted into a control signal for the means for varying the flow resistance (12), the rinsing pump (3) and/or the suction pump (11), according to a preset target capacity of the rinsing pump (3) and/or of the suction pump (11) for the operating position "on", and according to the target pressure, and vice versa.

2. The device according to claim 1, **characterized in that** the first medical instrument (5) is selected from the group consisting of "rinsing probe, trocar with optical system, optical system with rinsing channel and rinsing cannula".

3. The device according to claim 1 or 2, **characterized in that** the rinsing pump (3) comprises a drive unit with a motor having a rotating drive shaft and a pump unit, the capacity being controllable by controlling the speed of the motor, or that the rinsing pump (3) comprises an elastic storage container with a controllable pressure sleeve covering the storage container, the capacity being controllable by controlling the pressure sleeve, or that the rinsing pump (3) comprises a height-controllable storage container, the capacity being controllable by controlling the height of the storage container.

4. The device according to one of claims 1 to 3, **characterized in that** the second medical instrument (9) is selected from the group consisting of "shaver and suction probe".

5. The device according to one of claims 1 to 4, **characterized in that** the volume flow through the second medical instrument (9) in the operating position "on" is "high", and vice versa.

6. The device according to one of claims 1 to 5, **characterized in that** the means for varying the flow resistance (12) are controllable proportionally continuously or with a multitude of discrete steps.

7. The device according to one of claims 1 to 6, **characterized in that** the means for varying the flow resistance (12) are a hose clamping device with a hose (14), the hose wall (15) of which is made of an elastic material, with a support surface (16), on which the hose (14) rests, and with a pressure piece (17), by means of which a pressure can be exerted on the hose wall (15) in the direction of the support surface (16).

8. The device according to one of claims 1 to 7, **characterized in that** the pressure piece (17) can be driven linearly.

9. The device according to claim 8, **characterized in that** the pressure piece (17) is connected by a spindle gear box (18) with an electromotor drive (19), preferably a stepper motor.

## Revendications

1. Dispositif de rinçage d'une cavité de corps (1) avec un fluide,
comprenant un conteneur de stockage (2) pour le fluide, comprenant une pompe de rinçage (3) commandable liée au conteneur de stockage (2), comprenant une conduite d'alimentation (4) liée à un côté de pression de la pompe de rinçage (3) et comprenant un premier instrument médical (5) lié à la conduite d'alimentation (4) et ayant un canal de rinçage, ce premier instrument médical (5) étant capable d'être introduit dans la cavité de corps (1), sur le côté de pression de la pompe de rinçage (3) un capteur de pression (6) pour déterminer une pression réelle étant prévu,
comprenant une canule de drainage (7) étant capable d'être introduite dans la cavité de corps (1) et ayant une conduite de drainage (8),
comprenant un deuxième instrument médical (9) étant capable d'être introduit dans la cavité de corps (1) et ayant une conduite d'aspiration (10), un débit volumique réglable à travers la cavité de corps (1) étant "haut" ou "bas", selon une position de fonctionnement "marche" ou "arrêt" du deuxième instrument médical (9),
la conduite de drainage (8) et la conduite d'aspiration (10} étant liées à une pompe d'aspiration (11),
et le capteur de pression étant lié entre la pompe de rinçage (3) et l'ouverture de sortie du premier instrument médical (5) à l'extérieur de la cavité de corps (1),
**caractérisé en ce**
**que** seulement la conduite de drainage (8) est pourvue de moyens commandables pour varier la résistance à l'écoulement (12),
**que** la pompe de rinçage (3), le capteur de pression (6), la pompe d'aspiration (11) et les moyens pour varier la résistance à l'écoulement (12) sont liés à des entrées ou sorties de commande d'une unité de commande (13),
dans lequel au moyen de l'unité de commande (13) un abaissement de la pression réelle mesurée au moyen du capteur de pression (6) à partir d'une pression théorique donnée peut être détecté lors de la commutation du deuxième instrument médical (9) à partir de la position de fonctionnement "arrêt" à "marche" et peut être converti en un signal de commande pour les moyens pour varier la résistance à l'écoulement (12), la pompe de rinçage (3) et/ou la pompe d'aspiration (11), selon une capacité théorique spécifiée de la pompe de rinçage (3) et/ou de la pompe d'aspiration (11) pour la position de fonctionnement "marche", et selon la pression théorique, et inversement.

2. Dispositif selon la revendication 1, **caractérisé en ce que** le premier instrument médical (5) est choisi à partir du groupe consistant en "sonde de rinçage, trocart avec système optique, système optique avec canal de rinçage et canule de rinçage".

3. Dispositif selon la revendication 1 ou 2, **caractérisé en ce que** la pompe de rinçage (3) comprend une unité d'entraînement avec un moteur ayant un arbre d'entraînement tournant et une unité de pompe, la capacité étant commandable par commande de la vitesse du moteur, ou que la pompe de rinçage (3) comprend un conteneur de stockage élastique avec un brassard à pression commandable recouvrant le conteneur de stockage, la capacité étant commandable par commande du brassard à pression, ou que la pompe de rinçage (3) comprend un conteneur de stockage commandable en hauteur, la capacité étant commandable par commande de la hauteur du conteneur de stockage.

4. Dispositif selon une des revendications 1 à 3, **caractérisé en ce que** le deuxième instrument médical (9) est choisi à partir du groupe consistant en "shaver et sonde d'aspiration".

5. Dispositif selon une des revendications 1 à 4, **caractérisé en ce que** le débit volumique à travers le deuxième instrument médical (9) dans la position de fonctionnement "marche" est "haut", et inversement.

6. Dispositif selon une des revendications 1 à 5, **caractérisé en ce que** les moyens pour varier la résistance à l'écoulement (12) sont commandables proportionnellement continûment ou avec une multitude d'étapes discrètes.

7. Dispositif selon une des revendications 1 à 6, **caractérisé en ce que** les moyens pour varier le résistance à l'écoulement (12) sont un dispositif de serrage de tuyau avec un tuyau (14), la paroi (15) du tuyau étant en un matériau élastique, avec une surface de support (16), sur laquelle le tuyau (14) repose, et avec une pièce de pression (17), au moyen de laquelle une pression peut être exercée sur la paroi (15) du tuyau dans la direction de la surface de support (16) .

8. Dispositif selon une des revendications 1 à 7, **caractérisé en ce que** la pièce de pression (17) peut être entraînée linéairement.

9. Dispositif selon la revendication 8, **caractérisé en ce que** la pièce de pression (17) est liée par un engrenage à broche (18) à un entraînement électromoteur (19), de préférence un moteur pas à pas.
